# EUROPEAN PATENT SPECIFICATION

(11) **EP 3 373 905 B1**
(45) Date of publication and mention of the grant of the patent: **01.12.2021**
(21) Application number: 15908082.9
(22) Date of filing: 13.11.2015
(51) Int. Cl.: A61K 8/99, A61K 8/21, A61K 8/19, A61Q 11/00, A61K 8/73

(54) **DENTIFRICE COMPOSITIONS WITH IMPROVED CONSUMER EXPERIENCE**
ZAHNPASTAZUSAMMENSETZUNGEN MIT VERBESSERTER VERBRAUCHERERFAHRUNG
COMPOSITIONS DE DENTIFRICE OFFRANT AU CONSOMMATEUR UNE EXPÉRIENCE AMÉLIORÉE

(43) Date of publication of application: 19.09.2018
(73) Proprietor: The Procter & Gamble Company, Cincinnati, OH 45202 (US)
(72) Inventor: BASA, Swapna, Beijing 101312 (CN); STRAND, Ross, Singapore138547 (SG)
(74) Representative: P&G Patent Germany
(86) International application number: PCT/CN2015/094504
(87) International publication number: WO 2017/079952

(56) References cited:
- WO-A1-2009/134657
- WO-A1-2009/134657
- WO-A1-2015/094154
- WO-A1-2015/172344
- WO-A1-2015/172345
- WO-A1-2015/172347
- WO-A1-2015/172354
- GB-A- 1 132 830
- US-B1- 6 855 325

## Description

### FIELD OF THE INVENTION

The present invention relates to dentifrice compositions.

### BACKGROUND OF THE INVENTION

Dentifrice compositions are well known for dental and oral hygiene care. High water (e.g., >44 wt%) and high carbonate (e.g., >24 wt%) formulation chassis are a cost effective for many markets and consumers. For an optimal consumer experience, dentifrice composition should have a desirable viscosity. A classical guide to this viscosity range is from 150 Pa.s to 850 Pa.s (150000 to 850000 centipoise ("cP")). Of course, dentifrice composition must be physically stable, i.e., phase stable, over time. Additional considerations for a consumer preferred dentifrice composition experience include how easy the composition is dispensed on a toothbrush ("easiness to dispense"), the ability of the composition to spread on a toothbrush ("spread-ability"), or the foam amount (i.e., more foam is generally more desirable). The use of thickening agents, which may influence these consumer preferences of any dentifrice, are generally known art. However, there are myriad of such agents and their effect on consumer preferences, much less on any specific formulation chassis, is unpredictable. For example, WO2009/134657A1 discloses a thickening system comprising thickening silica, CMC and carrageenan in high-water and high calcium carbonate compositions or WO2015/094154A uses a combination of CMC and xanthan gum in similar compositions, wherein the latter also comprises 5 to 25wt% of glycerin humectant which diminishes the fluoride uptake.

Accordingly, there is a need for a high water, high carbonate, dentifrice compositions that have a consumer acceptable viscosity, that are physically stable over time, have a good fluoride uptake and that demonstrate at least one desirable consumer experience as to easiness to dispense, spreadability, or higher foaming amount.

### SUMMARY OF THE INVENTION

The present invention addresses at least one of these needs based on the surprising discovery of use of a thickening system as claimed in claim 1 in high water, high carbonate, dentifrice compositions that have a consumer acceptable viscosity, that are physically stable over time, and that demonstrate at least one desirable consumer experience as to easiness to dispense, spreadability, or higher foaming amount. Accordingly, one aspect of the invention provides a dentifrice composition as claimed in claim 1 Another aspect of the invention provides for a method of treating tooth enamel comprising the step of brushing teeth with the aforementioned dentifrice composition as claimed in claim 1.

While the specification concludes with claims that particularly point out and distinctly claim the invention, it is believed the present invention will be better understood from the following description.

### DETAILED DESCRIPTION OF THE INVENTION

### Definitions

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dentifrice" as used herein means paste, gel, powder, tablets, or liquid formulations, unless otherwise specified, that are used to clean the surfaces of the oral cavity. Preferably the dentifrice compositions of the present invention are single phase compositions. The term "teeth" as used herein refers to natural teeth as well as artificial teeth or dental prosthesis.

All percentages, parts and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt%" herein. All molecular weights as used herein are weight average molecular weights expressed as grams/mole, unless otherwise specified.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of" and "consisting essentially of'.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

### Water

The dentifrice compositions of the present invention comprise herein from 45% to 75%, by weight of the composition, of water. Preferably the dentifrice composition comprises from 45% to 65%, more preferably from 45% to 55%, yet more preferably from 46% to 54%, by weight of the composition, of water. The water may be added to the formulation and/or may come into the composition from the inclusion of other ingredients. Preferably the water is USP water.

### Calcium-containing abrasive

The compositions of the present invention comprise from 25% to 50% by weight of a calcium-containing abrasive, wherein preferably the calcium-containing abrasive is selected from the group consisting of calcium carbonate, calcium glycerophosphate, dicalcium phosphate, tricalcium phosphate, calcium orthophosphate, calcium metaphosphate, calcium polyphosphate, calcium oxyapatite, sodium carbonate, and combinations thereof. Preferably, the composition comprises from 26% to 47%, more preferably from 27% to 47%, even more preferably from 27% to 39%, yet even more preferably from 28% to 38%, alternatively from 29% to 37%, alternatively from 29% to 36%, alternatively from 30% to 35%, by weight of the composition, alternatively combinations thereof, of a calcium-containing abrasive.

Preferably the calcium-containing abrasive is calcium carbonate. More preferably, the calcium-containing abrasive is selected from the group consisting of fine ground natural chalk, ground calcium carbonate, precipitated calcium carbonate, and combinations thereof.

Fine ground natural chalk (FGNC) is one of the more preferred calcium-containing abrasives useful in the present invention. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling. One example of natural chalk is described in WO 03/030850 having a medium particle size of 1 to 15 µm and a BET surface area of 0.5 to 3 m²/g. The natural calcium carbonate may have a particle size of 325 to 800 mesh, alternatively a mess selected from 325, 400 600, 800, or combinations thereof; alternatively the particle size is from 0.1 to 30 microns, or from 0.1 to 20 microns, or from 5 to 20 microns

In one embodiment, the composition of the present invention comprises from 0 wt% to 5 wt%, preferably from 0 wt% to 3%, by weight of the composition, of silicate; alternatively the composition is free or substantially free of silicate.

### Thickening System

The dentifrice compositions comprise a thickening system, wherein the thickening system comprises at least a natural gum. In turn, the natural gum comprises from 0.2% to 1.8% by weight of the composition. The natural gum is xanthan gum. Xanthan gum is a polysaccharide secreted by the bacterium *Xanthomonas camestris.* Generally, xanthan gum is composed of a pentasaccharide repeat units, comprising glucose, mannose, and glucuronic acid in a molar ratio of 2:2:1, respectively. The chemical formula (of the monomer) is C₃₅H₄₉O₂₉.

The thickening system further comprises a thickening polymer (in addition to the natural gum) and a thickening silica.

The thickening polymer is selected from at least two, of the following thickening polymers: carboxymethyl cellulose ("CMC") and linear sulfated polysaccharide, wherein the CMC comprises from 0.01% to 3%, preferably 0.1% to 2.5%, more preferably 0.2% to 1.5% by weight of the dentifrice composition. The linear sulfated polysaccharide comprises from 0.01% to 2.5%, preferably 0.05 % to 2%, more preferably 0.1% to 1.5%, alternatively 0.1% to 1.3%, by weight of the dentifrice composition, wherein the linear sulfated polysaccharide is a carrageenan (also known as carrageenin). Examples of carrageenan include Kappa-carrageenan, Iota-carrageenan, Lambdacarrageenan, and combinations thereof.

The thickening system further comprises thickening silica. The thickening silica is from 0.01% to 10%, preferably from 0.1% to 9%, more preferably 1% to 8% by weight of the dentifrice composition. Alternatively the dentifrice composition comprises from 1.5% to 3.5%, by weight of the dentifrice composition, of the thickening silica, alternatively from 2% to 3%, alternatively from 2% to 5% alternatively from 1% to 3%, alternatively combinations thereof.

In one example the thickening silica is obtained from sodium silicate solution by destabilizing with acid as to yield very fine particles. One commercially available example is ZEODENT® branded silicas from Huber Engineered Materials (e.g., ZEODENT® 103, 124, 113 115, 163, 165, 167).

In one example the CMC is prepared from cellulose by treatment with alkali and monochloroacetic acid or its sodium salt. Different varieties are commercially characterized by viscosity. One commercially available example is Aqualon™ branded CMC from Ashland Special Ingredients (e.g., Aqualon™ 7H3SF; Aqualon™ 9M3SF Aqualon™ TM9A; Aqualon™ TM12A).

In one example, the xanthan gum is from CP Kelco Inc (Okmulgee, US).

### pH

The pH of the dentifrice composition is from pH 9.0 to pH 10.5, alternatively from pH 9 to pH 10. The relatively high pH of the present inventive composition is for fluoride stability. Without wishing to be bound theory, at below pH 8 calcium ion may bind with the fluoride. Thus, it is desirable to have the dentifrice composition have a greater than pH 8.0 to maximize the stability of the fluoride ion source. A method for assessing pH of dentifrice is described is provided the analytical methods section provided below. For purposes of clarification, although the analytical method describes testing the dentifrice composition when freshly prepared, for purposes of claiming the present invention, the pH may be taken at anytime during the product's reasonable lifecycle (including but not limited to the time the product is purchased from a store and brought to the consumer's home).

### pH modifying agent

The dentifrice compositions herein may include an effective amount of a pH modifying agent, alternatively wherein the pH modifying agent is a pH buffering agent. pH modifying agents, as used herein, refer to agents that can be used to adjust the pH of the dentifrice compositions to the above-identified pH range. pH modifying agents may include alkali metal hydroxides, ammonium hydroxide, organic ammonium compounds, carbonates, sesquicarbonates, borates, silicates, phosphates, imidazole, and mixtures thereof. Specific pH agents include monosodium phosphate (monobasic sodium phosphate or "MSP"), trisodium phosphate (sodium phosphate tribasic dodecahydrate or "TSP"), sodium benzoate, benzoic acid, sodium hydroxide, potassium hydroxide, alkali metal carbonate salts, sodium carbonate, imidazole, pyrophosphate salts, sodium gluconate, lactic acid, sodium lactate, citric acid, sodium citrate, phosphoric acid. In one embodiment, 0.01% to 3%, preferably from 0.1% to 1%, by weight of the composition, of TSP, and 0.001% to 2%, preferably from 0.01% to 0.3%, by weight of the composition, of monosodium phosphate is used. Without wishing to be bound by theory, TSP and monosodium phosphate may also have calcium ion chelating activity and therefore provide some monofluorophosphate stabilization (in those formulations containing monofluorophosphate).

### PEG

The compositions of the present invention may comprise polyethylene glycol (PEG), of various weight percentages of the composition as well as various ranges of average molecular weights. In one aspect of the invention, the compositions have from 0.01% to 8%, preferably from 0.1% to 5%, more preferably from 0.2% to 4.8%, yet more preferably from 0.3% to 4.2%, yet still more preferably from 0.5% to 4%, by weight of the composition, of PEG. In another aspect of the invention, the PEG is one having a range of average molecular weight from 100 Daltons to 1600 Daltons, preferably from 200 to 1000, alternatively from 400 to 800, alternatively from 500 to 700 Daltons, alternatively combinations thereof. PEG is a water soluble linear polymer formed by the addition reaction of ethylene oxide to an ethylene glycol equivalent having the general formula: H-(OCH₂CH₂)ₙ-OH. One supplier of PEG is Dow Chemical Company under the brandname of CARBOWAX™. Without wishing to be bound by theory, having some PEG in the dentifrice composition may help with physical stability.

### Sweetener

The oral care compositions herein may include a sweetening agent. These sweetener agents may include saccharin, dextrose, sucrose, lactose, maltose, levulose, aspartame, sodium cyclamate, D-tryptophan, dihydrochalcones, acesulfame, sucralose, neotame, and mixtures thereof. Sweetening agents are generally used in oral compositions at levels of from 0.005% to 5%, by weight of the composition, alternatively 0.01% to 1%, alternatively from 0.1% to 0.5%, alternatively combinations thereof.

### Fluoride ion source

The compositions may include an effective amount of an anti-caries agent. In one embodiment, the anti-caries agent is a fluoride ion source. The fluoride ion may be present in an amount sufficient to give a fluoride ion concentration in the composition at 25° C, and/or in one embodiment can be used at levels of from 0.0025% to 5% by weight of the composition, alternatively from 0.005% to 2.0% by weight of the composition, to provide anti-caries effectiveness. Representative fluoride ion sources include: stannous fluoride, sodium fluoride, potassium fluoride, amine fluoride, sodium monofluorophosphate, and zinc fluoride. In one embodiment the dentifrice composition contains a fluoride source selected from stannous fluoride, sodium fluoride, and mixtures thereof. In one embodiment, the fluoride ion source is sodium monofluorophosphate, and wherein the composition comprises 0.0025% to 2%, by weight of the composition, of the sodium monofluorophosphate, alternatively from 0.5% to 1.5%, alternatively from 0.6% to 1.7%, alternatively combinations thereof. In another embodiment, the composition comprises from 0.0025% to 2%, by weight of the composition, of a fluoride ion source. In one example, the dentifrice compositions of the present invention may have a dual fluoride ion source, specifically sodium monofluorophosphate and an alkaline metal fluoride. Such an approach may provide an improvement in mean fluoride update.

### Anti-calculus agent

The dentifrice compositions may include an effective amount of an anti-calculus agent, which in one embodiment may be present from 0.05% to 50%, by weight of the composition, alternatively from 0.05% to 25%, alternatively from 0.1% to 15% by weight of the composition. Non-limiting examples include those described in US 2011/0104081 A1 at paragraph 64, and those described in US 2012/0014883 A1 at paragraphs 63 to 68, as well as the references cited therein. One example is a pyrophosphate salt as a source of pyrophosphate ion. In one embodiment, the composition comprises tetrasodium pyrophosphate (TSPP) or disodium pyrophosphate or combinations thereof, preferably 0.01% to 2%, more preferably from 0.1% to 1%, by weight of the composition, of the pyrophosphate salt. Without wishing to be bound by theory, TSPP may provide not only calcium chelating thereby mitigating plaque formation, but may also provide the additional benefit of monofluorophosphate stabilization (in those formulations containing monofluorophosphate).

### Surfactant

The dentifrice compositions herein may include a surfactant. The surfactant may be selected from anionic, nonionic, amphoteric, zwitterionic, cationic surfactants, or mixtures thereof. The composition may include a surfactant at a level of from 0.1% to 10%, from 0.025% to 9%, from 0.05% to 5%, from 0.1% to 2.5%, from 0.5% to 2%, or from 0.1% to 1% by weight of the total composition. Non-limiting examples of anionic surfactants may include those described at US 2012/0082630 A1 at paragraphs 32, 33, 34, and 35. Non-limiting examples of zwitterionic or amphoteric surfactants may include those described at US 2012/0082630 A1 at paragraph 36; cationic surfactants may include those described at paragraphs 37 of the reference; and nonionic surfactants may include those described at paragraph 38 of the reference. In one embodiment the composition comprises 0.1% to 5%, preferably 0.1% to 3%, alternatively from 0.3% to 3%, alternatively from 1.2% to 2.4%, alternatively from 1.2% to 1.8%, alternatively from 1.5 % to 1.8%, by weight of the composition, alternatively combinations thereof, of the anionic surfactant sodium lauryl sulfate (SLS).

### Low or Free Humectants

The compositions herein may be substantially free or free of humectants, and contain low levels of humectants. The term "humectant," for the purposes of present invention, include edible polyhydric alcohols such as glycerin, sorbitol, xylitol, butylene glycol, propylene glycol, and combinations thereof. In one embodiment, the humectant is a polyol, preferably wherein the polyol is selected from sorbitol, glycerin, and combinations thereof. In yet another embodiment, the humectant is sorbitol. In one embodiment, the composition comprises from 0% to less than 5%, by weight of the composition, of humectants, preferably from 0% to 4%, alternatively from 0% to 3%, alternatively from 0% to 2%, alternatively from 0% to 1%, by weight of the composition, of humectants. The dentifrice compositions of the present invention comprise from 0% to 3%, more preferably 0% to 1%, by weight of the composition, of glycerin, sorbitol, or combinations thereof; yet more preferably the composition is substantially free of both glycerin and sorbitol.

A potential advantage of having a dentifrice composition that is free or substantially free of humectants is, without wishing to be bound by theory, is those dentifrice compositions that are free of polyols (e.g., glycerin and sorbitol), or have a relatively low amount thereof, may provide better fluoride uptake compared to those compositions having the high levels of such polyols (or humectants for that matter).

### Colorant

The compositions herein may include a colorant. Titanium dioxide is one example of a colorant. Titanium dioxide is a white powder which adds opacity to the compositions. Titanium dioxide generally can comprise from 0.25% to 5%, by weight of the composition.

### Flavorant

The compositions herein may include from 0.001% to about 5%, alternatively from 0.01% to 4%, alternatively from 0.1% to 3%, alternatively from 0.5% to 2%, alternatively 1% to 1.5% alternatively 0.5% to 1%, by weight of the composition, alternatively combinations thereof, of a flavorant composition. The term flavorant composition is used in the broadest sense to include flavor ingredients, or sensates, or sensate agents, or combinations thereof. Flavor ingredients may include those described in US 2012/0082630 A1 at paragraph 39; and sensates and sensate ingredients may include those described at paragraphs 40 - 45, incorporated herein by reference. Excluded from the definition of flavorant composition is "sweetener" (as described above).

### DATA

### Analytical Methods

A method for assessing pH of dentifrice is described. pH is measured by a pH Meter with Automatic Temperature Compensating (ATC) probe. The pH Meter is capable of reading to 0.001 pH unit. The pH electrode may be selected from one of the following (i) Orion Ross Sure-Flow combination: Glass body - VWR #34104-834/Orion #8172BN or VWR#10010-772/Orion #8172BNWP; Epoxy body - VWR #34104-830/Orion #8165BN or VWR#10010-770/Orion #8165BNWP; Semi-micro, epoxy body - VWR #34104-837/Orion #8175BN or VWR#10010-774/Orion #3175BNWP; or (ii) Orion PerpHect combination:VWR #34104-843/Orion #8203BN semi-micro, glass body; or (iii) suitable equivalent. The automatic temperature compensating probe is Fisher Scientific, Cat #13-620-16.

A 25% by weight slurry of dentifrice is prepared with deionized water, and thereafter is centrifuged for 10 minutes at 15000 rotations-per-minute using a SORVALL RC 28S centrifuge and SS-34 rotor (or equivalent gravitational force, at 24149g force). The pH is assessed in supernatant after one minute. After each pH assessment, the electrode is washed with deionized water. Any excess water is wiped with a laboratory grade tissue. When not in issue, the electrode is kept immersed in a pH 7 buffer solution or an appropriate electrode storage solution.

The method for assessing viscosity is described. The viscometer is Brookfield® viscometer, Model DV-I Prime with a Brookfield "Helipath" stand. The viscometer is placed on the Helipath stand and leveled via spirit levels. The E spindle is attached, and the viscometer is set to 2.5 RPM. Detach the spindle, zero the viscometer and install the E spindle. Then, lower the spindle until the crosspiece is partially submerged in the paste before starting the measurement. Simultaneously turn on the power switch on the viscometer and the helipath to start rotation of the spindle downward. Set a timer for 48 seconds and turn the timer on at the same time as the motor and helipath. Take a reading after the 48 seconds. The reading is in cP.

The term "phase stability" means visually (i.e., to the unaided eye) having no liquid separated from the oral care composition (e.g., toothpaste) body over a defined period of time under ambient conditions. In other words, a phase stable composition will resist syneresis. The data is provided at 2 years.

### Examples

Thirty five samples of dentifrice formulation are prepared and tested for viscosity, Phase stability, and pH. The variable tested is the thickening system. But for the thickening system, all of the dentifrice compositions tested include: 0.08 % monosodium phosphate; 0.42 % trisodium phosphate; 0.6% tetrasodium pyro-phosphate; 1.1% sodium monofluorophosphate; 0.25% sodium saccharin; 32% calcium carbonate (specifically FGNC); 0.85% mint flavorant (from Arvamint®); 7.5% sodium lauryl sulfate; and 2% of polyethylene glycol (combination of PEG 600 and PEG 12). The water level varied from 46% to 54% in these thirty five samples (depending upon the amount of the thickening system). The data is provided in Table 1 below.

**Table 1: Viscosity, Phase stability, and pH assessed in various dentifrice formulations.**

| **No.** | **Weight Percent in Dentifrice Composition** | | | | | | | |
|---|---|---|---|---|---|---|---|---|
| | **CMC (%)** | **Carrageenan (%)** | **X-gum (%)** | **Total Polymer (%)** | **Thicken ing Silica (%)** | **Viscosity (cP)** | **Phase Stability** | **pH** |
| 1 | 0.25 | 0 | 1.6 | 1.85 | 6 | 664000 | Yes | 9.21 |
| 2 | 0.75 | 0.1 | 1.6 | 2.45 | 1.5 | 524000 | Yes | 9.69 |
| 3 | 0.75 | 0.8 | 1 | 2.55 | 0.5 | 624000 | Yes | 9.89 |
| 4 | 0 | 0.3 | 1 | 1.3 | 1.5 | 224000 | Yes | 9.84 |
| 5 | 0.25 | 1.1 | 0.2 | 1.55 | 1 | 172000 | Yes | 10.02 |
| 6 | 0.25 | 1.3 | 0.8 | 2.35 | 2 | 624000 | Yes | 9.52 |
| 7 | 0 | 0.6 | 1 | 1.6 | 5.5 | 702000 | Yes | 9.21 |
| 8 | 0.5 | 0.7 | 1.2 | 2.4 | 4 | 846000 | Yes | 9.42 |
| 9 | 0.75 | 0.2 | 0.6 | 1.55 | 0 | 222000 | Yes | 10.29 |
| 10 | 0.25 | 0.8 | 1 | 2.05 | 0.5 | 446000 | Yes | 10.07 |
| 11 | 0.5 | 0.7 | 0.2 | 1.4 | 3 | 306000 | Yes | 9.55 |
| 12 | 1 | 0.1 | 0.2 | 1.3 | 6 | 782000 | Yes | 9.2 |
| 13 | 0 | 0.2 | 1.8 | 2 | 2.5 | 494000 | Yes | 9.4 |
| 13 | 0 | 0.6 | 0.2 | 0.8 | 6 | 352000 | Yes | 9.13 |
| 14 | 1.25 | 0.2 | 1 | 2.45 | 1.5 | 550000 | Yes | 9.81 |
| 15 | 0.25 | 0.5 | 1.8 | 2.55 | 1.5 | 738000 | Yes | 9.51 |
| 16 | 0.75 | 0.3 | 0.8 | 1.85 | 3 | 430000 | Yes | 9.34 |
| 17 | 0.25 | 1.1 | 0.6 | 1.95 | 3.5 | 604000 | Yes | 9.28 |
| 18^{∗} | 0.25 | 0.1 | 0.8 | 1.15 | 3.5 | 248000 | Yes | 9.43 |
| 19† | 0.91 | 1.2 | 0 | 2.11 | 2.62 | 618000 | Yes | 9.45 |

| Samples 20-35 fail to meet viscosity range or instability: | | | | | | | | |
|---|---|---|---|---|---|---|---|---|
| 20 | 0.75 | 0.2 | 0 | 0.95 | 2 | 90000 | No | 9.85 |
| 21 | 0.25 | 0.1 | 0 | 0.35 | 6 | 310000 | No | 9.12 |
| 22 | 0.75 | 0.2 | 0 | 0.95 | 2 | 90000 | No | 9.85 |
| 23 | 0 | 0.2 | 0.2 | 0.4 | 1.5 | 14000 | No | 9.81 |
| 24 | 1.25 | 1.1 | 0.2 | 2.55 | 1.5 | 372000 | No | 9.8 |
| 25 | 1.5 | 0.1 | 0.2 | 1.8 | 2 | 368000 | No | 9.79 |
| 26 | 1 | 0.7 | 0.8 | 2.5 | 6 | 1200000 | Yes | 9.2 |
| 27 | 0.5 | 0.3 | 1.8 | 2.6 | 6 | 1160000 | Yes | 9.36 |
| 28 | 0.5 | 0.6 | 0.4 | 1.5 | 7 | 1060000 | Yes | 9 |
| 29 | 0 | 0.2 | 0.2 | 0.4 | 1.5 | 14000 | No | 9.81 |
| 30 | 0 | 0.9 | 1.8 | 2.7 | 6 | 1830000 | Yes | 9.08 |
| 31 | 1 | 0.2 | 0.8 | 2 | 6 | 1120000 | Yes | 9.22 |
| 32 | 1.5 | 0.2 | 0.8 | 2.5 | 6 | 1570000 | Yes | 9.46 |
| 33 | 0.25 | 0.7 | 0.2 | 1.15 | 0.5 | 60000 | Yes | 10.21 |
| 34 | 0.5 | 1.2 | 0.2 | 1.9 | 6 | 1360000 | Yes | 9.15 |
| 35 | 1.5 | 0.3 | 0.2 | 2 | 6.5 | 1470000 | Yes | 9.18 |

Referring to Table 1 above, the following definitions are herein provided.
^{∗} Sample 18 is within the scope of the present invention, notably containing 0.8 wt% of xanthan gum.
† Sample 19 is not within the scope of the present invention. There is no xanthan gum. Although the sample has acceptable viscosity and stability, it is further tested by an expert consumer sensory panel as described below.

"CMC" means carboxymethyl cellulose and is from Ashland (China) Holdings CO Ltd (Shanghai CN)

"Carrageenan" is from FMC Biopolymer Co (Philadelphia, US).

"X-gum" is xanthan gum and is from CP Kelco Us Inc (Okmulgee, US)

"Total Polymer" is the weight percentage total of CMC, Carrageenan, and X-gum.

"Silica" is amorphous slilica gel from Zhaoqing Jinsanjiang Chemical Company Ltd.

"Viscosity" is at 28 days and per the analytical method described earlier. A viscosity of 150000 cP to 850000 cP is a classic viscosity target range for a consumer acceptable dentifrice.

"Phase Stability" is per the analytical method described earlier, and after 2 years under ambient conditions. The test is binary. Either "yes" the composition is phase stable, or "no" the composition is not phase stable.

"pH" is initial pH and per the analytical method described above.

Samples 1-18 are non-limiting examples that are within the scope of the present invention as having an acceptable viscosity and being physical stable over time. These samples notably all contain xanthan gum. Sample 19, although having an acceptable viscosity and being physically stable over time, is not within the scope of the invention because the sample does not contain xanthan gum. Sample 19 is a comparative example used in subsequent expert panelist testing described in detail below. Samples 20-35 are not within the scope of the invention as failing to meet the target viscosity range (i.e., between 150000 to 850000 cP) and/or physically unstable (after two years).

Samples 18 and 19 are tested by an expert sensory panel of 12 members.

**Table 2: Expert panelists assessing consumer experience comparing samples 18 and 19.**

| **Consumer Experience:** | **Sample 18 (Inventive)** | **Sample 19 (Comparative** | **Parity** | **Significant @ 90%** | **Result** |
|---|---|---|---|---|---|
| Easiness to Dispense | 12 | 0 | 0 | Yes | #18 is easier to dispense |
| Spread -ability | 12 | 0 | 0 | Yes | #18 is easier to spread |
| Luster | 7 | 5 | 0 | No | -- |
| Foam amount | 8 | 2 | 2 | Yes | #18 has higher foam amount |

The data from table 2 indicate that although both samples 18 and 19 are stable and within consumer acceptable viscosity ranges; however inventive sample 18 provides a better consumer experience than comparative sample 19. Specifically, inventive sample 18 is easier to both dispense on a toothbrush and spread on the toothbrush. Furthermore, sample 18 provides a higher foam amount during brushing. Although not statistically significant, there is a suggestion that the sample 18 may have a more desirable luster appearance. For any one of these reasons, inventive sample 18 (having xanthan gum) provides a superior consumer experience than comparative example 19 (without xanthan gum).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

## Claims

1. A dentifrice composition comprising:
(a) 45% to 75%, by weight of the composition, of water;
(b) 25% to 50%, by weight of the composition, of a calcium-containing abrasive;
(c) 0.0025% to 2%, by weight of the composition, of a fluoride ion source;
(d) a thickening system comprising:
at least a natural gum, wherein the natural gum comprises from 0.2% to 1.8% by weight of the composition and wherein the natural gum is xanthan gum, and
further comprises a thickening polymer, wherein the thickening polymer is from 0.01% to 3% of carboxymethyl cellulose and 0.01% to 2.5% of a linear sulfated polysaccharide, wherein the linear sulfated polysaccharide is a carrageenan, and
a thickening silica, wherein the thickening silica is from 0.01% to 10%; and
wherein the composition comprises from 0% to 3% by weight of the composition, of glycerin, sorbitol, or combinations thereof; and
wherein the pH is from 9 to 10.5.

2. The dentifrice composition according to any one of the preceding claims, wherein the thickening polymer is selected from:
(a) 0.1% to 2.5% of a carboxymethyl cellulose by weight of the composition, preferably 0.2% to 1.5%, by weight of the composition, of CMC; and
(b) 0.05 % to 2%, preferably 0.1% to 1.5% of carrageenan.

3. The dentifrice composition according to any one of the preceding claims, wherein the thickening polymer, including the natural gum, is from 0.5% to 4%, preferably from 0.8% to 3.5%, more preferably from 1% to 3%, yet still more preferably from 1.3% to 2.6% by weight of the composition.

4. The dentifrice composition according to any one of the preceding claims, wherein the thickening silica is from 0.1% to 9%, preferably from 1% to 8% by weight of the composition.

5. The dentifrice composition according to any one of the preceding claims, wherein the water is from 45% to 55% by weight of the composition; and preferably wherein the calcium-containing abrasive is calcium carbonate.

6. The dentifrice composition according to anyone of the preceding claims, wherein the calcium-containing abrasive is 27% to 47%, preferably 27% to 37%, by weight of the composition, and more preferably wherein the calcium-containing abrasive is a calcium carbonate.

7. The dentifrice composition according to anyone of the preceding claims, wherein the fluoride ion source is sodium monofluorophosphate, preferably wherein the sodium monofluorophosphate is from 0.5% to 1.5% by weight of the composition.

8. The dentifrice composition according to any one of the preceding claims, further comprising 0.1% to 12%, by weight of the composition, of a surfactant, preferably 1% to 9% of the surfactant, more preferably wherein the surfactant is an anionic surfactant, yet more preferably wherein the anionic surfactant is sodium lauryl sulfate.

9. The dentifrice composition according to any one of the preceding claims, further comprising a polyethylene glycol, preferably the composition comprises from 0.1% to 5%, by weight of the composition, of the PEG, more preferably from 0.5% to 4%, yet more preferably from 1% to 3%, by weight of the composition, of PEG.

10. The dentifrice composition according to any one of the preceding claims, wherein the composition comprises from 0% to 1%, by weight of the composition, of glycerin, sorbitol, or combinations thereof; preferably the composition is free of both glycerin and sorbitol.

## Patentansprüche

1. Zahnpastazusammensetzung, umfassend:
(a) zu 45 Gew.-% bis 75 Gew.-% der Zusammensetzung Wasser;
(b) zu 25 Gew.-% bis 50 Gew.-% der Zusammensetzung ein calciumhaltiges Schleifmittel;
(c) zu 0,0025 Gew.-% bis 2 Gew.-% der Zusammensetzung eine Fluoridionenquelle;
(d) ein Verdickungssystem, umfassend:
mindestens einen natürlichen Gummi, wobei der natürliche Gummi von 0,2 Gew.-% bis 1,8 Gew.-% der Zusammensetzung ausmacht und wobei der natürliche Gummi Xanthangummi ist, und
ferner ein Verdickungspolymer umfasst, wobei das Verdickungspolymer von 0,01 % bis 3 % Carboxymethylcellulose und 0,01 % bis 2,5 % ein lineares sulfatiertes Polysaccharid ist, wobei das lineare sulfatierte Polysaccharid ein Carrageenan ist, und
ein verdickendes Silica, wobei das verdickende Silica von 0,01 % bis 10 % beträgt; und
wobei die Zusammensetzung von 0 Gew.-% bis 3 Gew.-% der Zusammensetzung Glycerin, Sorbit oder Kombinationen davon umfasst; und
wobei der pH-Wert von 9 bis 10,5 beträgt.

2. Zahnpastazusammensetzung nach einem der vorstehenden Ansprüche, wobei das Verdickungspolymer ausgewählt ist aus:
(a) 0,1 Gew.-% bis 2,5 Gew.-% der Zusammensetzung eine Carboxymethylcellulose, vorzugsweise 0,2 Gew.-% bis 1,5 Gew.-% der Zusammensetzung CMC; und
(b) 0,05 % bis 2 %, vorzugsweise 0,1 % bis 1,5 %, Carrageenan.

3. Zahnpastazusammensetzung nach einem der vorstehenden Ansprüche, wobei das Verdickungspolymer, einschließlich des natürlichen Gummis, von 0,5 Gew.-% bis 4 Gew.-%, vorzugsweise von 0,8 Gew.-% bis 3,5 Gew.-%, mehr bevorzugt von 1 Gew.-% bis 3 Gew.-%, noch mehr bevorzugt von 1,3 Gew.-% bis 2,6 Gew.-% der Zusammensetzung beträgt.

4. Zahnpastazusammensetzung nach einem der vorstehenden Ansprüche, wobei das verdickende Silica von 0,1 Gew.-% bis 9 Gew.-%, vorzugsweise von 1 Gew.-% bis 8 Gew.-% der Zusammensetzung beträgt.

5. Zahnpastazusammensetzung nach einem der vorstehenden Ansprüche, wobei das Wasser von 45 Gew.-% bis 55 Gew.-% der Zusammensetzung beträgt; und wobei vorzugsweise das calciumhaltige Schleifmittel Calciumcarbonat ist.

6. Zahnpastazusammensetzung nach einem der vorstehenden Ansprüche, wobei das calciumhaltige Schleifmittel 27 Gew.-% bis 47 Gew.-%, vorzugsweise 27 Gew.-% bis 37 Gew.-% der Zusammensetzung beträgt und wobei mehr bevorzugt das calciumhaltige Schleifmittel ein Calciumcarbonat ist.

7. Zahnpastazusammensetzung nach einem der vorstehenden Ansprüche, wobei die Fluoridionenquelle Natriummonofluorphosphat ist, wobei das Natriummonofluorphosphat vorzugsweise von 0,5 Gew.-% bis 1,5 Gew.-% der Zusammensetzung beträgt.

8. Zahnpastazusammensetzung nach einem der vorstehenden Ansprüche, ferner umfassend zu 0,1 Gew.-% bis 12 Gew.-% der Zusammensetzung ein Tensid, vorzugsweise zu 1 Gew.-% bis 9 Gew.-% das Tensid, wobei das Tensid mehr bevorzugt ein anionisches Tensid ist, wobei das anionische Tensid noch mehr bevorzugt Natriumlaurylsulfat ist.

9. Zahnpastazusammensetzung nach einem der vorstehenden Ansprüche, ferner umfassend ein Polyethylenglycol, wobei die Zusammensetzung vorzugsweise zu von 0,1 Gew.-% bis 5 Gew.-% der Zusammensetzung das PEG, mehr bevorzugt zu von 0,5 Gew.-% bis 4 Gew.-%, noch mehr bevorzugt zu von 1 Gew.-% bis 3 Gew.-% der Zusammensetzung PEG umfasst.

10. Zahnpastazusammensetzung nach einem der vorstehenden Ansprüche, wobei die Zusammensetzung zu von 0 Gew.-% bis 1 Gew-% der Zusammensetzung Glycerin, Sorbit oder Kombinationen davon umfasst; wobei die Zusammensetzung vorzugsweise frei von sowohl Glycerin als auch Sorbit ist.

## Revendications

1. Composition dentifrice comprenant :
(a) 45 % à 75 %, en poids de la composition, d'eau ;
(b) 25 % à 50 %, en poids de la composition, d'un abrasif contenant du calcium ;
(c) 0,0025 % à 2 %, en poids de la composition, d'une source d'ions fluorure ;
(d) un système épaississant comprenant :
au moins une gomme naturelle, dans laquelle la gomme naturelle constitue de 0,2 % à 1,8 % en poids de la composition et dans laquelle la gomme naturelle est de la gomme de xanthane, et
comprend en outre un polymère épaississant, dans laquelle le polymère épaississant est de 0,01 % à 3 % de carboxyméthylcellulose et 0,01 % à 2,5 % d'un polysaccharide sulfaté linéaire, dans laquelle le polysaccharide sulfaté linéaire est un carraghénane, et
une silice épaississante, dans laquelle la silice épaississante va de 0,01 % à 10 % ; et
dans laquelle la composition comprend de 0 % à 3 % en poids de la composition, de glycérine, de sorbitol, ou de combinaisons de ceux-ci ; et
dans laquelle le pH va de 9 à 10,5.

2. Composition dentifrice selon l'une quelconque des revendications précédentes, dans laquelle le polymère épaississant est choisi parmi :
(a) 0,1 % à 2,5 % d'une carboxyméthylcellulose en poids de la composition, de préférence 0,2 % à 1,5 %, en poids de la composition, de CMC ; et
(b) 0,05 % à 2 %, de préférence 0,1 % à 1,5 % de carraghénane.

3. Composition dentifrice selon l'une quelconque des revendications précédentes, dans laquelle le polymère épaississant, incluant la gomme naturelle, est de 0,5 % à 4 %, de préférence de 0,8 % à 3,5 %, plus préférablement de 1 % à 3 %, même encore plus préférablement de 1,3 % à 2,6 % en poids de la composition.

4. Composition dentifrice selon l'une quelconque des revendications précédentes, dans laquelle la silice épaississante est de 0,1 % à 9 %, de préférence de 1 % à 8 % en poids de la composition.

5. Composition dentifrice selon l'une quelconque des revendications précédentes, dans laquelle l'eau est de 45 % à 55 % en poids de la composition ; et de préférence dans laquelle l'abrasif contenant du calcium est du carbonate de calcium.

6. Composition dentifrice selon l'une quelconque des revendications précédentes, dans laquelle l'abrasif contenant du calcium est de 27 % à 47 %, de préférence 27 % à 37 %, en poids de la composition, et plus préférablement dans laquelle l'abrasif contenant du calcium est un carbonate de calcium.

7. Composition dentifrice selon l'une quelconque des revendications précédentes, dans laquelle la source d'ions fluorure est du monofluorophosphate de sodium, de préférence dans laquelle le monofluorophosphate de sodium est de 0,5 % à 1,5 % en poids de la composition.

8. Composition dentifrice selon l'une quelconque des revendications précédentes, comprenant en outre 0,1 % à 12 % d'un agent tensioactif en poids de la composition, de préférence 1 % à 9 % de l'agent tensioactif, plus préférablement dans laquelle l'agent tensioactif est un agent tensioactif anionique, encore plus préférablement dans laquelle l'agent tensioactif anionique est du laurylsulfate de sodium.

9. Composition dentifrice selon l'une quelconque des revendications précédentes, comprenant en outre un polyéthylène glycol, de préférence la composition comprend de 0,1 % à 5 %, en poids de la composition, du PEG, plus préférablement de 0,5 % à 4 %, encore plus préférablement de 1 % à 3 %, en poids de la composition, de PEG.

10. Composition dentifrice selon l'une quelconque des revendications précédentes, dans laquelle la composition comprend de 0 % à 1 %, en poids de la composition, de glycérine, de sorbitol, ou de combinaisons de ceux-ci ; de préférence la composition est exempte à la fois de glycérine et de sorbitol.
